# EUROPÄISCHE PATENTSCHRIFT

(11) **EP 0 452 909 B1**
(45) Veröffentlichungstag und Bekanntmachung des Hinweises auf die Patenterteilung: **03.01.1996**
(21) Anmeldenummer: 91106154.7
(22) Anmeldetag: 17.04.1991
(51) Int. Cl.: C07C 17/38, C07C 19/045, C07C 21/06

(54) **Verfahren zur Reinigung von nicht umgesetztem 1,2-Dichlorethan aus einem 1,2-Dichlorethan-Pyrolyseprozess**
Method of purifying non-converted 1,2-dichloroethane from a 1,2-dichloroethane pyrolysis process
Procédé d'épuration d'1,2-dichloroéthane non-converti d'un procédé de pyrolyse d'1,2-dichloroéthane

(30) Priorität: 19.04.1990 DE 4012538
(43) Veröffentlichungstag der Anmeldung: 23.10.1991
(73) Patentinhaber: WACKER-CHEMIE GMBH, D-81737 München (DE)
(72) Erfinder: Schmidhammer, Ludwig, Dr., W-8261 Haiming (DE); Haselwarter, Klaus, W-8261 Emmerting (DE); Klaus, Herrmann, W-8261 Marktl (DE); Mohr, Klaus-Peter, W-8263 Burghausen (DE)

(56) Entgegenhaltungen:
- EP-A- 0 002 501
- EP-A- 0 180 998
- DD-A- 139 839
- DE-A- 2 445 371
- DE-A- 4 033 047
- FR-A- 2 038 347

## Beschreibung

Die Erfindung betrifft ein Verfahren zur Reinigung von nicht umgesetztem 1,2-Dichlorethan aus einem 1,2-Dichlorethan-Pyrolyseprozeß durch Chlorierung des als Nebenprodukt anfallenden Benzols und destillative Entfernung der Chlorierungsprodukte.

Bei der unvollständigen pyrolytischen Zerlegung von 1,2-Dichlorethan in Vinylchlorid und Chlorwasserstoff entsteht unter anderem auch Benzol, das sich bei der nachfolgenden destillativen Aufarbeitung dieses Crackgasgemisches (HCl-Entfernung in der HCl-Kolonne, VC- und EDC-Trennung in der nachfolgenden VC-Kolonne und schließlich Entfernung von leicht- und hochsiedenden Verunreinigungen aus dem nichtumgesetzten EDC in der nachgeschalteten Leicht- bzw. Hochsiederkolonne), aufgrund seines Siedepunktes, im Sumpfprodukt der Vinylchlorid-Kolonne, dem nicht umgesetzten 1,2-Dichlorethan, anreichert. Je nach Crackbedingungen bzw. Aufarbeitung des nicht umgesetzten 1,2-Dichlorethans zu reinem 1,2-Dichlorethan, das wieder in den Pyrolyseprozeß zurückgeführt werden kann, kommt es zu einer mehr oder weniger starken Benzolanreicherung im 1,2-Dichlorethan, wodurch die Kinetik des Pyrolyseprozeßes äußerst ungünstig beeinflußt wird.

In der Literatur (Ullmann 4. Auflage, Band 9 (1975), Seite 448) wird Benzol zwar nur als schwacher Inhibitor bei der thermischen Zerlegung von 1,2-Dichlorethan in Vinylchlorid und Chlorwasserstoff beschrieben, wobei dort definitionsgemäß der Inhibitoreffekt einer Substanz dann als schwach anzusehen ist, wenn Konzentrationen über 500 ppm im 1,2-Dichlorethan zugegen sein müssen, damit der Inhibitoreffekt signifikant nachweisbar ist.

Wird jedoch nach der Pyrolyse nicht umgesetztes 1,2-Dichlorethan im Kreislauf geführt und nach der Aufbereitung wieder der Pyrolyse zugeführt, können im zu spaltenden 1,2-Dichlorethan Benzolkonzentrationen bis zu 5000 Gewichtsppm und mehr auftreten, und die Inhibitorwirkung von Benzol tritt dann recht deutlich zu Tage. Diese Anreicherung von Benzol tritt vor allem dann auf, wenn das nicht umgesetzte 1,2-Dichlorethan nach der Pyrolyse gemäß dem Verfahren der EP-A 2501 (US-A 4188347) vorbehandelt wird (Nebenproduktentfernung durch einen Chlorierungs- und nachfolgenden Dechlorierungsschritt in Gegenwart hydroxylgruppenhaltiger Aromaten) und entsprechend der EP-A 180998 energiesparend aufbereitet wird, indem man nur einen kleinen Teilstrom des vorbehandelten nicht umgesetzten 1,2-Dichlorethans, vor der Zuführung zur Pyrolyseeinheit, zum Beispiel gemäß der DE-A 2445371 (US-A 3998706), von Leichtsiedern befreit.

Bei der Pyrolyse selbst nimmt zudem mit steigendem Umsatz auch der Gehalt von Benzol im Crackgasgemisch zu. Wird nun zur Kompensation des Inhibitoreffekts des Benzols die Pyrolysetemperatur erhöht, ergeben sich als Folge davon zwangsläufig erhöhte Spaltrohrwandtemperaturen. Diese wiederum führen zu einer verstärkten Ausbildung von Benzol und anderen Nebenprodukten, die vom gebildeten Vinylchlorid nur sehr schwer abtrennbar sind, aber die Polymerisation von Vinylchlorid äußerst negativ beeinflussen. Zudem führen die erhöhten Spaltrohrwandtemperaturen zu einem vermehrten Koksanfall. Durch diese erhöhten Koksablagerungen in den Spaltrohren reduzieren sich die Ofenlaufzeiten und damit auch die Anlagekapazitäten. Zusätzlich werden aufgrund der starken Wärmeisolierwirkung solcher Koksablagerungen noch weiter erhöhte Spaltrohrwandtemperaturen notwendig, um durch Erhöhung der Temperaturdifferenz zwischen Rohrwand und Produkt die Isolierwirkung des Kokses zu kompensieren. Ganz allgemein fördern erhöhte Wandtemperaturen zudem auch die Hochtemperaturkorrosion des Spaltrohrmaterials durch Chlorwasserstoffangriff, wodurch die Standzeiten der Spaltrohre vermindert werden (erhöhte Instandhaltungskosten) bzw. im ungünstigsten Falle sogar äußerst gefährliche Betriebszustände durch Rohrreißer eintreten können (Gefahr für Personal, Anlage und Umwelt).

Das Problem der Benzolanreicherung hat sich in jüngster Zeit stark in den Vordergrund gestellt, weil inzwischen energiesparende Vinylchloridherstellverfahren bei möglichst hohen Anlagendurchsätzen, wie zum Beispiel die ebengenannten Verfahrensweisen zur Aufbereitung von nicht umgesetztem 1,2-Dichlorethan, immer mehr angestrebt werden. Dadurch kommt es aber zwangsweise zu einer verstärkten Anreicherung von Benzol in 1,2-Dichlorethan.

Eine rein destillative Benzolabtrennung aus 1,2-Dichlorethan scheidet aus wirtschaftlichen Gründen von vorneherein aus, weil dazu aufgrund der sehr nahe beieinanderliegenden Siedepunkte ein großer technischer wie energetischer Aufwand erforderlich wäre.

Aus der Literatur ist wohlbekannt, daß man Benzol in Gegenwart katalytisch wirkender Halogenüberträger in weitaus höher siedende Chloraromaten umwandeln kann. Ein wohlfeiler Halogenüberträger ist beispielsweise Eisen-III-chlorid, das in wasserfreier, pulvriger bis feinkörniger Form verwendet wird, aber auch in situ durch Reaktion zwischen Chlor und metallischem Eisen erzeugt werden kann.

Letztere Verfahrensweise ist in der DE-PS 1493381 (US-A 3366457) beschrieben. In kontinuierlicher Fahrweise wird im Überschuß Benzol mit Chlor, in Gegenwart von metallischem Eisen, zu Monochlorbenzol umgesetzt. In der DE-PS 558068 wird ein Verfahren beschrieben bei dem im Überschuß Benzol mit Chlor, in Gegenwart von metallischem Eisen, in Form von Raschigringen und Eisendraht, in der Dampfphase zu Monochlorbenzol umgesetzt wird.

Diese Verfahrensweisen haben den Nachteil, daß zur Chlorierung von Benzol in starker Verdünnung (Benzol ist im nicht umgesetzten 1,2-Dichlorethan meist im Konzentrationsbereich von 1000 bis 8000 Gew.ppm vorhanden), zur Erzielung ausreichender Chlorumsätze bei moderaten Reaktionsbedingungen, massive Eisen-III-Chlorid-Konzentrationen von mehreren 1000 ppm, bezogen auf 1,2-Dichlorethan, erforderlich sind. Die Löslichkeit von wasserfreiem FeCl₃ in trockenem 1,2-Dichlorethan mit einem Wassergehalt von weniger als 10 Gewppm beträgt je nach Einwirkdauer jedoch nur etwa 350 bis 600 Gewppm; ist also nicht ausreichend.

Aus der DE-A 1568298 (GB-A 1184576) ist bekannt, durch Verwendung von Eisen-III-Oxid anstelle von metallischem Eisen und/oder Eisen-III-Chlorid die Löslichkeit von FeCl₃ in 1,2-Dichlorethan etwa um den Faktor 10 zu erhöhen. Der Grund dafür ist das, durch die Reaktion zwischen Eisen-III-Oxid und Chlorwasserstoff,sich bildende Wasser, welches offensichtlich als starker Lösungsvermittler fungiert. In der NL-A 7215333 wird gelehrt mit 200 ppm Wasser versetztes Benzol zur Chlorierung von Benzol unter Verwendung von metallischem Eisen einzusetzen. Nachteilig bei diesen Verfahrensweisen ist jedoch, daß durch die Anwesenheit von Wasser das Reaktionsmedium stark korrosiv wird, was eine teure korrosionsfeste Ausführung der verwendeten Apparaturen erforderlich macht.

Aufgabe der Erfindung war es, ein Verfahren zur Reinigung von nicht umgesetztem 1,2-Dichlorethan aus einem 1,2-Dichlorethan-Pyrolyseprozeß auszuarbeiten, wodurch die Abtrennung von Benzol aus 1,2-Dichlorethan mit möglichst einfachen Mitteln erfolgt und die Anreicherung von Benzol in 1,2-Dichlorethan auf Konzentrationen größer 500 Gewppm verhindert wird.

Überraschenderweise ist es gelungen, durch Chlorierung bei moderaten Reaktionsbedingungen und unter Anwendung minimaler Chlorüberschüsse, bezogen auf den Benzolgehalt im nichtumgesetzten 1,2-Dichlorethan, in Gegenwart von feinverteiltem Eisenkatalysator, der den gesamten Reaktionsraum ausfüllt, einen fast vollständigen Benzolumsatz zu den entsprechenden Chlorierungsprodukten zu erzielen, welche dann leicht destillativ abgetrennt werden können. Dies ist umso überraschender, als bei dieser Verfahrensweise die Konzentration an katalytisch aktivem Eisen-III-Chlorid, das sich dabei ins 1,2-Dichlorethan hineinlöst, auch nur bei 350 bis 600 Gewppm liegt.

Gegenstand der Erfindung ist ein Verfahren zur Reinigung von nicht umgesetztem 1,2-Dichlorethan, das bei der Herstellung von Vinylchlorid durch unvollständige Pyrolyse von 1,2-Dichlorethan im Sumpf der Vinylchloridkolonne anfällt, dadurch gekennzeichnet, daß
a) bei der 1,2-Dichlorethan-Pyrolyse zurückgewonnenes, nicht umgesetztes 1,2-Dichlorethan in einem Chlorierungsreaktor in Flüssigphase, bei einer Verweilzeit von 20 bis 150 Minuten, bei Temperaturen von 20 bis 80°C, in Gegenwart von, den gesamten Reaktionsraum ausfüllendem, Eisenkatalysator mit einer Schüttdichte von 0.1 bis 0.5 g/cm³, mit flüssigem und/oder gasförmigem Chlor in solchen Mengen in Kontakt gebracht wird, daß das den Reaktor verlassende, nicht umgesetzte 1,2-Dichlorethan überschüssiges, freies Chlor in gelöster Form in Konzentrationen von 20 bis 200 Gewppm, bezogen auf nicht umgesetztes 1,2-Dichlorethan, enthält, und
b) das gemäß a) mit Chlor behandelte 1,2-Dichlorethan durch Sauer- und Alkaliwäsche, sowie durch destillative Leichtsieder- und Hochsiederabtrennung gereinigt wird.

Das erfindungsgemäße Verfahren zur Reinigung von bei der Pyrolyse nicht umgesetztem 1,2-Dichlorethan wird, nach der Entfernung von Chlorwasserstoff und Vinylchlorid aus dem Spaltgas-Gemisch der 1,2-Dichlorethan-Pyrolyse, mit dem Sumpfprodukt der Vinylchlorid-Kolonne durchgeführt. Das Sumpfprodukt wird in einen Reaktor übergeführt, wobei die Strömungsgeschwindigkeit im Reaktor 0.01 bis 0.1 cm/s, vorzugsweise 0.03 bis 0.06 cm/s, jeweils auf den freien Reaktorquerschnitt bezogen, beträgt. Die Verweilzeit im Reaktor beträgt 20 bis 150 Minuten, vorzugsweise 40 bis 80 Minuten, jeweils bezogen auf den leeren Reaktorraum.

Vor Eintritt in den Reaktor oder auch erst im Reaktor, vorzugsweise vor Eintritt in den Reaktor, wird dem 1,2-Dichlorethan-Strom flüssiges und/oder gasförmiges Chlor zugemischt. In jedem Fall sollte der 1,2-Dichlorethan-Strom mit solchen Mengen an Chlor in Konktakt gebracht werden, daß der den Reaktor verlassende 1,2-Dichlorethan-Strom überschüssiges freies Chlor, in gelöster Form, in Konzentrationen von 20 bis 200 Gewppm, vorzugsweise 50 bis 100 Gewppm, bezogen auf 1,2-Dichlorethan, enthält.

Die Chlorierungsreaktion erfolgt im Reaktor in Flüssigphase, bei Temperaturen von 20 bis 80°C, vorzugsweise 30 bis 50°C.

Der gesamte Reaktionsraum ist dabei mit feinverteiltem Eisenkatalysator mit einer Schüttdichte von 0.1 bis 0.5 g/cm³, vorzugsweise von 0.2 bis 0.3 g/cm³, ausgefüllt. Als Katalysatoren eignen sich insbesonders feinverteilte Eisenspäne, Eisenflechtwerk und/oder Stahlwolle. Prinzipiell kann auch Eisenflechtwerk oder Stahlwolle in feinstverteilter Form, das heißt mit Schüttdichten kleiner 0.1 g/cm³ verwendet werden. Bei kontinuierlichen Durchflußverfahren ist dabei jedoch die Gefahr der Reaktorverlegung nicht auszuschließen.

Das erfindungsgemäße Verfahren wird vorzugsweise vollkontinuierlich betrieben, kann jedoch auch absatzweise kontinuierlich erfolgen, wenn beispielsweise die Benzolanreicherung aufgrund besonderer Randbedingungen nur relativ gering ist.

Nach der Chlorbehandlung wird die 1,2-Dichlorethanphase von dem gebildeten Eisenchlorid und Chlorwasserstoff durch Sauer- und Alkaliwäsche befreit. Die Sauerwäsche kann in herkömmlicher Weise mittels verdünnter Salzsäure erfolgen; die Alkaliwäsche mit wäßrigem Ammoniak oder verdünnter Natronlauge. Nach der destillativen Abtrennung von Wasser und leichtsiedenden Verunreinigungen wird die 1,2-Dichlorethanphase einer Hochsiederkolonne zugeführt, in der die Abtrennung von hochsiedenden Verunreinigungen, inklusive der Chloraromaten, die bei der Benzolchlorierung entstanden sind, erfolgt. Das so aufbereitete 1,2-Dichlorethan kann dann wieder dem Pyrolyseprozeß zugeführt werden.

In einer besonders bevorzugten Ausführungsform des erfindungsgemäßen Verfahrens wird das Spaltproduktgemisch aus der 1,2-Dichlorethanspaltung nach der Entfernung von Chlorwasserstoff und Vinylchlorid zunächst, analog der Verfahrensweise in der EP-A 2501, mit 0.0001 bis 0.01 Gew%, bezogen auf nicht umgesetztes 1,2-Dichlorethan, hydroxylgruppenhaltiger Aromaten versetzt, und einem Chlorierungs- und nachfolgendem Dechlorierungsschritt bei Temperaturen zwischen 0 und 80°C und Drücken von 0.5 bis 6 bar unterzogen. Nur ein kleiner Teilstrom von vorzugsweise bis zu 20 Gew%, der so behandelten 1,2-Dichlorethanphase, wird dann entsprechend dem erfindungsgemäßen Verfahren mit Chlor behandelt und danach durch Sauer- und Alkaliwäsche gereinigt. Dieser Teilstrom wird dann zusammen mit rohem 1,2-Dichlorethan aus der Direkt- und/oder Oxychlorierung von Ethylen, vorzugsweise analog der Verfahrensweise gemäß der DE-A 2445371, von Leichtsiedern befreit und der Hochsiederkolonne zugeführt. Der Hauptstrom, der gemäß EP-A 2501 vorbehandelten 1,2-Dichlorethanphase, vorzugsweise mindestens 80 Gew%, wird unter Umgehung der Chlorierungsstufe, der Wäscher und der Leichtsiederabtrennung, direkt der Hochsiederkolonne zugeführt.

Durch die erfindungsgemäße Verfahrensweise gelingt es den Benzolgehalt im reinen 1,2-Dichlorethan, das am Kopf der Hochsiederkolonne abgezogen wird und der 1,2-Dichlorethan-Pyrolyse zugeführt wird, bei Werten kleiner als 500 Gewppm zu halten.

Die nachfolgenden Beispiele dienen zur weiteren Erläuterung der erfindungsgemäßen Verfahrensweise.

### Beispiel 1:

Der Reaktor bestand aus einem senkrecht stehenden Glasrohr (Innendurchmesser: 2.05 cm; Höhe: 100 cm; Volumen: 330 cm³), das mit einem Mantel umgeben war, in dem thermostatisiertes Wasser bei 30 bzw. 50°C im Kreis gepumpt wurde. Die gesamte Glasapparatur war braun eingefärbt und zusätzlich mit Alufolie umwickelt, um durch Licht initiierte Photochlorierungseffekte auszuschließen. Sämtliche Belüftungsöffnungen waren mit Molsiebtrockenröhrchen versehen, um das Eindringen von Luftfeuchtigkeit zu verhindern. Chlorgas wurde über ein mit Quarzwolle gefülltes und als Mischer wirkendes verdunkeltes Glasrohr dem mit 2500 Gew.ppm Benzol versetzten 1,2-Dichlorethan zugegeben. Die Chlorgasmessung erfolgte über einen geeichten Blasenzähler mit Hexachlorbutadien als Sperrflüssigkeit. Die Dosierung der Flüssigkeit zum Mischer erfolgte über eine geeichte Tropfvorrichtung, die dosierte Menge betrug 360 cm³/h. Das Reaktionsrohr war in unterschiedlicher Schütthöhe mit Stahlwolle der Fa. STAX, Qualität Fein, Werkstoff Nr. 1.0313, mit einer Schüttdichte von etwa 0.22 g/cm³ gefüllt. Das flüssige, mit freiem Chlor unterschiedlicher Konzentrationen beladene,ReaktionsgemiSch wurde nach Verlassen des Reaktionsraumes in eine Vorlage geleitet, in der abwechselnd eine wäßrige Kaliumiodidlösung für die jodometrische Chlorbestimmung mit 0.01 M Natriumthiosulfatlösung bzw. eine 0.01 M Salzsäure für die komplexometrische Eisen-III-Chlorid-Bestimmung nach Extraktion aus der organischen Phase eingefüllt war. Die organische Phase wurde nach Abtrennung der wäßrigen Phase über Natriumsulfat wasserfrei getrocknet und anschließend gaschromatographisch auf Benzol, Mono-, Di- und Trichlorbenzol untersucht.

Die Ergebnisse der einzelnen Versuche sind in Tabelle 1 festgehalten.

Die Zahlen der Tabelle 1 lassen sich wie folgt interpretieren:
Benzolumsatz bei der Chlorierung in beiden Fällen größer 99.8 %; Verweilzeit bei Versuch A 55 Minuten, bei Versuch B 33 Minuten; Strömungsgeschwindigkeit in beiden Fällen bei 0.03 cm/s.

Die "Selektivität" der Benzolchlorierung in Abhängigkeit vom Chlorüberschuß ergibt sich wie folgt:

| | Benzolumsatz in Mol% zu | | |
|---|---|---|---|
| | Mono-, | Di-, | Trichlorbenzol |
| bei 180 Gewppm Cl₂-Überschuß | 60 | 30 | 10 |
| bei 90 Gewppm Cl₂-Überschuß | 90 | 7 | 3 |

Daraus ist ersichtlich, daß mit wachsendem Chlorüberschuß der spezifische Chlorverbrauch pro Mol Benzol stark ansteigt.

### Vergleichsbeispiel 1:

Die Versuche wurden analog zu Beispiel 1 durchgeführt, anstelle von Stahlwolle wurden jedoch Eisendrehspäne mit einer Schüttdichte von etwa 0.85 g/cm³ bzw. Eisen-III-Chlorid in einer Konzentration von 550 Gewppm verwendet.
Selbst bei einer Verweilzeit von 200 Minuten, 50°C Reaktionstemperatur und einem Chlorüberschuß von 300 Gewppm im Reaktionsprodukt nach Verlassen des Reaktionsraumes gemessen, in dem auch 500 bzw. 550 Gewppm Eisen-III-Chlorid analysiert wurden, betrug der Benzolumsatz nur 64 % bzw. 68 %.

### Beispiel 2:

Bei der thermischen Spaltung von 74.6 t/h 1,2-Dichlorethan zu 28.1 t/h Vinylchlorid und 16.5 t/h Chlorwasserstoff (59.8 % Umsatz) fielen im Sumpf der Vinylchloridkolonne unter einem Druck von 6 bar absolut 30 t/h nicht umgesetztes 1,2-Dichlorethan an, das mit 6 Gewppm 1,1,2-Trichlorethan, 50 Gewppm Ethylchlorid, 1500 Gewppm 2-Chlorbuta-1,3-dien, 150 Gewppm 1-Chlor-buta-1,3-dien und etwa 200 Gewppm überwiegend unbekannten Di-, Tri-,Tetrachlorierten Diolefinen mit 2 bis 6 Kohlenstoffatomen, sowie Spuren an Chlorbenzolen verunreinigt war. Nach Abkühlen auf 30°C wurde das nicht umgesetzte 1,2-Dichlorethan mit 10 Gewppm o-Kresol versetzt und durch Zugabe von 0.843 Kmol/h verdampftem Flüssigchlor bzw. von 0.1944 Kmol/h gasförmigem Ethylen (zur Entfernung des Chlorüberschusses nach der Chlorierungsstufe) von leichtsiedenden, chlorierbaren Verunreinigungen befreit.
Das so vorbehandelte bei der 1,2-Dichlorethan-Pyrolyse nicht umgesetzte 1,2-Dichlorethan enthielt jetzt 476 Gewppm 1,1,2-Trichlorethan, 50 Gewppm Ethylchlorid und 100 Gewppm 2,3-Dichlorbuta-1,3-dien, war also frei von 2-Chlorbuta-1,3-dien bzw. 1-Chlor-buta-1,3-dien und anderen chlorierten Diolefinen. Der Gehalt an freiem Chlor betrug 2 Gewppm, Eisen-III-chlorid war nur in Spuren (< 2 Gewppm) zugegen. Daneben waren infolge vorheriger wiederholter Kreislaufführung noch etwa 300 Gewppm Benzol vorhanden.
Um eine weitere Benzolanreicherung zu verhindern, wurden 3 t/h des so vorbehandelten nicht umgesetzten 1,2-Dichlorethans nun bei 30°C über einen zylindrischen Stahlreaktor geleitet, der folgende Maße hatte: Innerer Durchmesser 1250 mm, Höhe 2500 mm, Volumen 3.05 m³. Der Stahlzylinder war mit 3 m³ Stahlwolle der Fa. Stax, Qualität Fein, Werkstoff Nr. 1.0313, mit einer Schüttdichte von etwa 0.22 g/cm³ gefüllt. Vor Eintritt in den Reaktor wurden diesem Strom 760 Nl/h Gaschlor zugesetzt. Bei einer Verweilzeit von rund 75 Minuten und einer Strömungsgeschwindigkeit von etwa 0.055 cm/s hatte das den Chlorierungsreaktor verlassende 1,2-Dichlorethan folgende Zusammensetzung:
< 10 Gewppm Benzol
391 Gewppm Monochlorbenzol
45 Gewppm Dichlorbenzol
7 Gewppm Trichlorbenzol
532 Gewppm Eisen-III-Chlorid
80 Gewppm freies Chlor
506 Gewppm 1,1,2-Trichlorethan
50 Gewppm Ethylchlorid
< 10 Gewppm 2,3-Dichlorbuta-1,3-dien
Anschließend wurde das "kernchlorierte" nicht umgesetzte 1,2-Dichlorethan zusammen mit 45.5 t/h rohem 1,2-Dichlorethan aus der Direkt- und Oxychlorierung von Ethylen (Gewichtsverhältnis etwa 45:55) sauer und alkalisch gewaschen und zur Entwässerung unter gleichzeitiger Leichtsiederabtrennung einer Leichtsiederkolonne zugeführt. Das Sumpfprodukt dieser Leichtsiederkolonne wurde zusammen mit den restlichen 27 t/h chloroprenfreiem, aber noch 300 Gewppm Benzol enthaltendem, nicht umgesetztem 1,2-Dichlorethan einer Schwersiederkolonne zugeführt, wo am Kopf etwa 74 t/h reines 1,2-Dichlorethan abgezogen wurden, die rund 20 Gewppm Ethylchlorid enthielten, und frei von Eisen-III-Chlorid waren.
Obwohl dieses gereinigte 1,2-Dichlorethan einer unvollständigen Pyrolyse unterworfen wurde, die ständig Benzol nachlieferte, und das dabei nicht umgesetzte 1,2-Dichlorethan, entsprechend der in diesem Beispiel beschriebenen Verfahrensweise stetig im Kreis geführt wurde, konnte der Benzolpegel im 1,2-Dichlorethan, das dem Spaltofen zugeführt wurde, über einen Zeitraum von 4500 Stunden bei Werten kleiner 200 Gewppm gehalten werden. Die Ofenstandzeit bis zur nächsten Entkokung betrug 6 Monate.

### Vergleichsbeispiel 2:

Es wurde analog Beispiel 2 verfahren, jedoch ohne den erfindungsgemäßen Schritt der Benzolchlorierung. Dadurch ergab sich ein 1,2-Dichlorethan für die thermische Spaltung, dessen Benzolpegel im Verlauf von 3000 Stunden bis auf etwa 5000 Gewppm anstieg. Es konnte nur eine Ofenstandzeit von etwa 4 Monaten erzielt werden. Zur Erzielung eines vergleichbaren Umsatzes mußte die Produkttemperatur sukzessive von 495 auf 510°C (Temperatur am Coilende) angehoben werden.

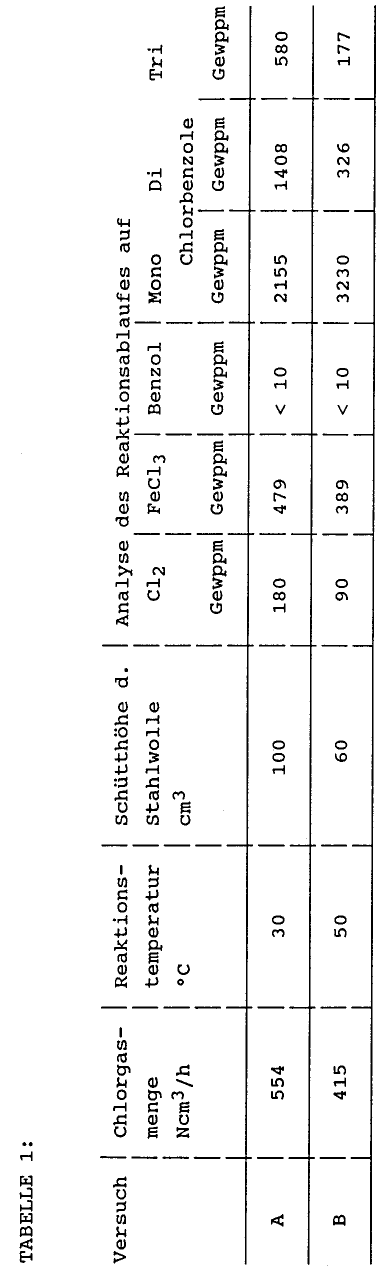

## Patentansprüche

1. Verfahren zur Reinigung von nicht umgesetztem 1,2-Dichlorethan, das bei der Herstellung von Vinylchlorid durch unvollständige Pyrolyse von 1,2-Dichlorethan im Sumpf der Vinylchloridkolonne anfällt, dadurch gekennzeichnet, daß
a) bei der 1,2-Dichlorethan-Pyrolyse zurückgewonnenes, nicht umgesetztes 1,2-Dichlorethan in einem Chlorierungsreaktor in Flüssigphase, bei einer Verweilzeit von 20 bis 150 Minuten, bei Temperaturen von 20 bis 80°C, in Gegenwart von, den gesamten Reaktionsraum ausfüllendem, Eisenkatalysator mit einer Schüttdichte von 0.1 bis 0.5 g/cm³, mit flüssigem und/oder gasförmigem Chlor in solchen Mengen in Kontakt gebracht wird, daß das den Reaktor verlassende, nicht umgesetzte 1,2-Dichlorethan überschüssiges, freies Chlor in gelöster Form in Konzentrationen von 20 bis 200 Gewppm, bezogen auf nicht umgesetztes 1,2-Dichlorethan, enthält, und
b) das gemäß a) mit Chlor behandelte 1,2-Dichlorethan durch Sauer- und Alkaliwäsche, sowie durch destillative Leichtsieder- und Hochsiederabtrennung gereinigt wird.

2. Verfahren zur Reinigung von nicht umgesetztem 1,2-Dichlorethan, das bei der Herstellung von Vinylchlorid durch unvollständige Pyrolyse von 1,2-Dichlorethan im Sumpf der Vinylchloridkolonne anfällt, dadurch gekennzeichnet, daß
das nicht umgesetzte 1,2-Dichlorethan zunächst mit 0.0001 bis 0.01 Gew%, bezogen auf nicht umgesetztes 1,2-Dichlorethan, hydroxylgruppenhaltiger Aromaten versetzt, und einem Chlorierungs- und nachfolgendem Dechlorierungsschritt bei Temperaturen zwischen 0 und 80°C und Drücken von 0.5 bis 6 bar unterzogen wird,
und nur ein Teilstrom, der so behandelten 1,2-Dichlorethanphase, dann gemäß Anspruch 1 mit Chlor behandelt und danach durch Sauer- und Alkaliwäsche gereinigt wird,
dieser Teilstrom dann zusammen mit rohem 1,2-Dichlorethan aus der Direkt- und/oder Oxychlorierung von Ethylen von Leichtsiedern befreit und der Hochsiederkolonne zugeführt wird,
und der Hauptstrom unter Umgehung der Chlorierungsstufe, der Wäscher und der Leichtsiederabtrennung, direkt der Hochsiederkolonne zugeführt wird.

3. Verfahren nach Anspruch 1 oder 2, dadurch gekennzeichnet, daß die Strömungsgeschwindigkeit des nicht umgesetzten 1,2-Dichlorethans zwischen 0.01 und 0.1 cm/s, bezogen auf den freien Reaktorquerschnitt, beträgt.

## Claims

1. Process for purifying unreacted 1,2-dichloroethane produced at the bottom of the vinyl chloride column in the preparation of vinyl chloride due to incomplete pyrolysis of 1,2-dichloroethane, characterized in that
a) unreacted 1,2-dichloroethane recovered from the pyrolysis of 1,2-dichloroethane is brought into contact with liquid and/or gaseous chlorine in a chlorination reactor in the liquid phase at a residence time of 20 to 150 minutes, at temperatures of from 20 to 80°C, in the presence of an iron catalyst having a bulk density of from 0.1 to 0.5 g/cm³ and filling the entire reaction space, in such amounts that the unreacted 1,2-dichloroethane leaving the reactor contains excess, free chlorine in dissolved form in concentrations of from 20 to 200 ppm by weight, based on unreacted 1,2-dichloroethane, and
b) the 1,2-dichloroethane treated with chlorine in accordance with a) is purified by acid and alkali washing and by distillative removal of the low- and high-boiling components.

2. Process for purifying unreacted 1,2-dichloroethane produced at the bottom of the vinyl chloride column in the preparation of vinyl chloride due to incomplete pyrolysis of 1,2-dichloroethane, characterized in that
the unreacted 1,2-dichloroethane is first treated with from 0.0001 to 0.01 % by weight, based on unreacted 1,2-dichloroethane, of hydroxyl-containing aromatic compounds and subjected to a chlorination step and subsequent dechlorination step at temperatures between 0 and 80°C and pressures of from 0.5 to 6 bar,
and then only a part stream of the 1,2-dichloroethane phase treated in this way is treated with chlorine as claimed in claim 1 and the part stream is then purified by acid and alkaline washing,
this part streams, together with crude 1,2-dichloroethane from the direct chlorination and/or oxychlorination of ethylene, is then freed from low-boiling components and passed to the high-boiling component column, and the main stream is passed directly to the high-boiling component column, avoiding the chlorination step, the washer and the low-boiling component removal step.

3. Process according to Claim 1 or 2, characterized in that the flow rate of the unreacted 1,2-dichloroethane is between 0.01 and 0.1 cm/s, based on the free reactor cross-section.

## Revendications

1. Procédé d'épuration d' 1,2-dichloroéthane non converti qui se dépose au fond de la colonne de chlorure de vinyle lors de la préparation du chlorure de vinyle par pyrolyse incomplète d' 1,2-dichloroéthane, caractérisé en ce que :
a) le 1,2-dichloroéthane non converti, récupéré de la pyrolyse du 1,2-dichloroéthane est mis en contact avec du chlore liquide et/ou gazeux, dans un réacteur de chloration en phase liquide, pour une durée de séjour de 20 à 150 minutes, à une température de 20 à 80°C, en présence d'un catalyseur au fer, remplissant complètement la chambre de réaction avec une épaisseur de couche de 0,1 à 0,5 g par cm³, en une quantité telle que le 1,2-dichloroéthane non converti, séparé du réacteur, contient du chlore libre en excès sous forme dissoute, en des concentrations de 20 à 200 ppm en poids, sur base du 1,2-dichloroéthane non converti, et
b) le 1,2-dichloroéthane traité par le chlore suivant a) est purifié par lavages acide et alcalin, ainsi que par séparation par distillation à ébullition faible et élevée.

2. Procédé d'épuration de 1,2-dichloroéthane non converti qui se dépose au fond de la colonne de chlorure de vinyle lors de la préparation du chlorure de vinyle par pyrolyse incomplète de 1,2-dichloroéthane, caractérisé en ce que l'on ajoute d'abord au 1,2-dichloroéthane non converti, 0,0001 à 0,01% en poids, sur base du 1,2-dichloroéthane non converti, un composé aromatique présentant des radicaux hydroxyle, en ce qu'on le soumet à une étape de chloration et à une étape de déchloration suivante à des températures entre 0 et 80°C et à des pressions de 0,5 à 6 bar et en ce qu'un courant partiel seulement de la phase 1,2-dichloroéthane ainsi traitée, est traité suivant la revendication 1 par le chlore et ensuite est purifié par lavages acide et alcalin,
ce courant partiel, avec le 1,2-dichloroéthane brut provenant de la chloration directe et/ou de l'oxychloration de l'éthylène, est alors libéré de l'ébullition faible et est amené vers la colonne d'ébullition élevée,
et le courant principal est amené par contournement de l'étape de chloration, du lavage et de la séparation à ébullition faible, directement à la colonne d'ébullition élevée.

3. Procédé suivant la revendication 1 ou 2, caractérisé en ce que la vitesse d'écoulement de 1,2-dichloroéthane non converti atteint entre 0,01 et 0,1 cm par seconde, sur base de la section transversale libre du réacteur.
